Europäisches Patentamt

European Patent Office

Office européen des brevets

(11) Publication number: **0 315 949**
**A2**

# EUROPEAN PATENT APPLICATION

(21) Application number: 88118575.5

(22) Date of filing: 08.11.88

(51) Int. Cl.⁴: **C12P 1/00** , //(C12P1/00, C12R1:40)

The applicant has filed a statement in accordance with Rule 28 (4) EPC (issue of a sample only to an expert). Accession number(s) of the deposit(s): FERM BP-1749, FERM BP-1750, FERM BP-1751.

(30) Priority: 09.11.87 JP 281130/87

(43) Date of publication of application:
17.05.89 Bulletin 89/20

(84) Designated Contracting States:
BE CH DE FR GB IT LI NL SE

(71) Applicant: **IDEMITSU KOSAN COMPANY LIMITED**
**No. 1-1, 3-chome, Marunouchi Chiyoda-ku Tokyo(JP)**

(72) Inventor: **Doi, Seiji**
**Idemitsu Kosan Company Limited 1280, Kamiizumi**
**Sodegaura-machi Kimitsu-gun Chiba-ken(JP)**
Inventor: **Horikoshi, Koki**
**39-8, 4-chome, Sakuradai Nerima-ku Tokyo(JP)**

(74) Representative: **Vossius & Partner**
**Siebertstrasse 4 P.O. Box 86 07 67 D-8000 München 86(DE)**

(54) A method for microbiological reaction.

(57) A method for producing a useful organic compound by a microbiological reaction from a less useful organic compound is described. In this method, the starting organic compound is brought into contact with a transformed microorganism obtained by introducing a plasmid carrying the genetic information for the desired reaction into a microorganism belonging to the genus Pseudomonas and having a resistance to hydrocarbons, alcohols, ethers and ketones by utilizing the techniques of genetic engineering. The microbiological reaction is performed in a two-phase system composed of an aqueous and organic phase or in an organic rare-water system. Types of the reactions to which the inventive method is applicable include oxidation reactions, reducing reactions, hydrolysis and transesterification reactions, isomerization and rearrangement reactions, lyase reactions and ligase reactions.

EP 0 315 949 A2

# A METHOD FOR MICROBIOLOGICAL REACTION

The present invention relates to a microbiological reaction or, more particularly, relates to a method for producing a useful organic compound by use of a microorganism obtained by the transformation of a starting microorganism having a resistance against organic solvents with a gene according to a method of genetic engineering.

Various methods are known in the prior art as reported in many literatures for producing a useful organic compound by bringing a microorganism into contact with a less useful starting organic compound including a method of contacting Norcadia sp. with hexane or hexadecane reported by R.L. Raymond in Appl. Microbiol., volume 15, pages 857-865 (1967), a method of contacting Bacterium JOB5 with cyclopentane or cyclohexane reported by J. Ooyama and J.W. Foster in Antonie-von Leenwenlook, volume 31, pages 45-65 (1965), or a method of contacting Pseudomonas sp., Achromobacter sp. or Norcadia sp. with benzene, ethyl benzene, toluene or xylene reported by D. Cleus and N. Walkes in J. Gen. Microbiol., volume 36, pages 107-122 (1964).

In these methods in which a hydrocarbon compound is brought into contact with a microorganism, the hydrocarbon compounds in general exhibit strong toxicity against the microorganisms so that the starting hydrocarbon compound must be supplied in the form of a vapor in order to avoid direct contacting of the liquid hydrocarbon compound and the microorganism as a dilution with an inert solvent in such a low concentration of, for example 0.2% or lower, that the microorganism is not affected by the toxicity of the hydrocarbon compound. Therefore, fermentative processes for industrial production by using a hydrocarbon compound as the substrate in general are practically not feasible due to the extremely low productivity and the difficulty in controlling the concentration of the substrate at a low level. Moreover, when a material hardly soluble in water is used, the productivity of the microbiological reaction is significantly decreased as a consequence of the low solubility of the material.

Accordingly the technical problem underlying the present invention is to provide a novel and improved method for microbiologically producing a useful compound by contacting a starting organic compound with a microorganism capable of growing even in a culture medium containing an organic solvent such as hydrocarbon compounds and the like in a high concentration, i.e. with a microorganism being resistant against organic solvents such as hydrocarbon compounds.

The solution of the above technical problem is achieved by providing a method which comprises contacting a starting organic compound with a microorganism obtained by the transformation of a starting microorganism belonging to the genus Pseudomonas and having a resistance against an organic compound selected from the group consisting of aliphatic hydrocarbon compounds, alicyclic hydrocarbon compounds, aromatic hydrocarbon compounds, alcohols, ethers and ketones by introducing a gene utilizing a method of genetic engineering.

In particular, the above mentioned starting microorganism belonging to the genus Pseudomonas preferably belongs to the species Pseudomonas putida or, and more preferably, it is the strain Pseudomonas putida var. STM-603 (FERM BP-1751).

The reaction of the method of the present invention is preferably carried out in a two-phase system composed of an aqueous phase and an organic phase of an organic solvent not freely miscible with water or in a rare-water system which means in an organic solvent containing a limited small amount of water dissolved therein.

The figure illustrates a flowchart showing the architectural method of plasmid RSF 2074.

As is described above, the invention relates to a microbiological reaction in which a starting organic compound is contacted with a specific microorganism belonging to the genus Pseudomonas and after its transformation with a gene utilizing a method of genetic engineering.

Examples of the host microorganism belonging to the genus Pseudomonas and having a resistance against the above mentioned organic compounds include Pseudomonas putida var. STM-603 (FERM P-9228, FERM BP-1751), Pseudomonas sp. STM-801 (FERM P-9226, FERM BP-1749) and Pseudomonas STM-904 (FERM P-9227, FERM BP-1750). The microbiological properties for the characterization of Pseudomonas putida var. STM-603 are described in detail in the specification of Japanese Patent Kokai 63-216473 and the strain is deposited at Fermentation Research Institute, Agency of Industrial Science,and Technology, under a deposit No. of FERM BP-1751. Similarly, the microbiological properties for the characterization of Pseudomonas sp. STM-801 and Pseudomonas sp. STM-904 are described in detail in the specification of Japanese Patent Kokai 63-216474 and the strains are each deposited at the Fermentation Research Institute as mentioned above under the deposit Nos. of FERM BP-1749 and FERM BP-1750, respectively.

The microbiological reaction of the inventive method is performed by using a microorganism obtained by a transformation of one of the above described microorganisms as the host in which a gene is introduced utilizing a method of genetic engineering. The type of the transformation for the preparation of such a transformed strain is appropriately selected in consideration of the type of the reaction involved in the inventive method. Types of the reaction which may take place in the inventive method include oxidation reactions such as epoxidation, hydroxylation or dehydrogenation; reducing reactions such as aldehyde reduction, ketone reduction or hydrogenation ; hydrolysis reactions and transesterification reactions in which esters, polysaccharides or peptides are hydrolyzed or transesterified; isomerization reactions such as isomerization relative to optical activity or cis-trans steric isomerization; rearrangement reactions in which glycosyl groups, acyl groups or phosphate groups are transferred within the same molecule; lyase reactions in which certain linkages such as C-C, C-O or C-N are eliminated as in the synthesis of L-tyrosine by utilizing the reverse reaction of β-tyrosinase to synthesize a C-C linkage; ligase reactions for the synthesis of various compounds having linkages such as C-C, C-O or C-N.

Accordingly, the transformed strain of the microorganisms used in the inventive method is obtained by the transformation of the above mentioned host microorganism into which a specific gene necessary for conducting either one of the above named reactions is introduced by utilizing a method of genetic engineering. For example, the above mentioned host microorganisms can be transformed by introducing a plasmid carrying at least one gene such as a hydrocarbon-decomposing plasmid, e.g. naphthalene-decomposing plasmid, toluene- decomposing plasmid or a camphor- and octane-decomposing plasmid or a gene encoding an enzyme for the oxidation of benzene by utilizing a known method of transformation of ordinary Pseudomonas microorganisms described in Curr. Top. Microbiol. Immunol., volume 96, page 47 (1981), a method of conjugative transfer or techniques of genetic recombination as described inmolecular Cloning published by Cold Spring Harber Laboratory (1982) and the thus obtained transformed microorganism is used for the oxidation reaction. Further, the host microorganism can be transformed by introducing a gene of β-tyrosinase utilizing the technique of genetic recombination and the thus transformed microorganism is used for the lyase reaction. As a further possibility, a DNA containing a gene corresponding to a diamino pimelic acid isomerization enzyme, such as the plasmid pDF3 described in J. Bacteriol., volume 169 (4), page 1454 (1987), is cleaved down with an appropriate restriction enzyme to separate the essential part and both ends of the thus obtained fragment of the gene are converted to the restriction ends formed by cleavage with a restriction enzyme suitable for the purpose, such as ECORI, while a plasmid vector, such as RSF1010, which is stable in the above mentioned microorganism having a resistance against organic solvents is cleaved to have the same restriction ends as the above mentioned fragment of the gene and the two gene fragments are combined together to give a recombinant plasmid which is introduced into the above mentioned resistant microorganism to cause transformation thereof. The thus transformed microorganisms can be used in an isomerization reaction as in the production of meso-form diamino pimelic acid according to the inventive method.

Besides, the resistant microorganism can be transformed similarly to the above manner by utilizing a plasmid containing a gene corresponding to the dehydrogenation enzyme of branched-chain keto acids, such as the plasmid pSS1-1 described in J. Bacteriol., volume 169 (4), page 1619 (1987), and the thus obtained transformed strain can be used in a reducing reaction as in the production of a branched-chain hydroxy acid. Similarly, DNAS containing a gene corresponding to the γ-glutamyl-L-cysteine synthesis enzyme GSH-1 or to the glutathione synthesis enzyme GSH-2, such as the plasmid pBR325-gsh I′•II described in "Genetic Engineering for Material Production", edited by The Agricultural Chemical Society of Japan, published by Asakura Shoten (1983), page 185, are used for introducing the genes into plasmid vector capable of being stably replicated in the resistant microorganism in the same manner as in the above described diamino pimelic acid followed by introduction of the same into the above mentioned resistant microorganism to create a glutathione-producing microorganism which is utilized in a ligase reaction, for example, for the synthesis of glutathione. In addition, a DNA containing a gene corresponding to the serine-hydroxymethyl transferase SHTase, such as the plasmid pBR322-glyA described in Preprint for General Meeting of The Agricultural Chemical Society of Japan (1985), page 29, can be introduced into the resistant microorganism by a method similar to the above to create a serin-producing microorganism and the thus transformed strain is used in a rearrangement reaction for the production of serine and the like by the intramolecular transfer of the hydroxymethyl group.

In addition, the above described microorganisms having a resistance against organic solvents can be used in hydrolysis reactions including hydrolysis and transesterification of esters. It is also possible to use the above described microorganisms by utilizing their characteristic resistance against organic solvents in the production of various kinds of fat-soluble substances such as fat-soluble vitamins, fat-soluble antibiotics, fat-soluble hormones and the like.

As is understood from the above description, the method of the present invention is applicable to a variety of reactions. The reactions can be classified into the reactions in a proliferating system and the reactions in resting microorganisms. The reactions of the former type can be further classified into reactions of fermentative production in which useful materials are produced by a complex metabolic system and conversion reactions in which a relatively simple enzymatic reaction or enzyme system is used. The reactions of the latter type are all conversion reactions. The reactions can be classified relative to the type of the reaction medium into ordinary aqueous system reactions, in which the reaction is performed in a medium mainly composed of water such as buffer solutions; two-phase system reactions, in which the reaction medium is composed of an aqueous phase and an organic phase of a solvent not freely miscible with water; and rare-water system reactions, in which the reaction medium is composed of an organic solvent containing a small amount of water dissolved therein within the limit of solubility so that the microorganism can be supplied with water necessary for existence, i.e. dehydration is not performed. The inventive method utilizes the reactions of the latter two types excepting the aqueous phase reactions.

As is described before, the host microorganism used in the inventive method should have resistance against one kind or two kinds or more of organic solvents. In particular, no microorganisms belonging to the genus Pseudomonas are known which have resistance against two kinds or more of organic solvents. Examples of the organic solvents, against which the host microorganism should have resistance, include aliphatic hydrocarbon compounds such as pentane, hexane, heptane, octane, isooctane, nonane, decane, 1- and 2-hexenes, 1-octene, 1-dodecene, 1,3-pentadiene, 1,5-hexadiene or 1,7-octadiene ; alicyclic hydrocarbon compounds such as cyclopentane, cyclohexane, methyl cyclopentane or methyl cyclohexane; aromatic hydrocarbon compounds such as toluene, xylene, styrene, ethyl benzene or chlorobenzene; alcohols such as heptan-1-ol, octan-1-ol or decan-1-ol; ethers such as n-hexyl ether, n-butyl phenyl ether, diphenyl ether, dibenzyl ether or methoxy toluene and ketones such as pentan-2-one, hexan-2-one, heptan-2-one or cyclohexanone.

The organic compounds used as the starting material in the method of the invention and contacted with the above mentioned transformed microorganisms include various kinds of water-soluble and fat-soluble ones. Examples of usable organic compounds include, in addition to the above named organic solvents exemplified by aliphatic hydrocarbon compounds , organic acids such as pyruvic acid, lactic acid or fumaric acid; esters such as ethyl acetate, butyl acetate or octyl acetate; saccharides; amino acids; nucleic acids; steroids and terpenoids as well as other nitrogen- or sulfur-containing organic compounds. In some cases, the reaction medium is admixed with ammonia or another suitable inorganic compound in addition to the above mentioned organic compound as in the production of L-tyrosine.

The culture medium used for culturing the microorganism used in the inventive method is not particularly limitative provided that proliferation of the microorganism can actively proceed therein. Examples of suitable carbon source materials include saccharides, e.g., glucose, sucrose, xylose, starch and hydrolysis products of starch; alcohols, e.g., methyl alcohol and ethyl alcohol; aromatic hydrocarbon compounds, e.g., benzene, toluene and xylene; and organic acids, e.g., succinic acid, salicylic acid and toluic acid. Examples of suitable nitrogen source materials include meat extract, peptone, yeast extract, dry yeast, corn steep liquor, Casamino acid, urea, ammonium chloride, ammonium nitrate, sodium nitrate or ammonium sulfate. The culture medium is usually admixed with various kinds of inorganic salts including phosphates and salts of metals such as phosphorus, magnesium, calcium, iron, potassium, copper or manganese, each in a small amount.

When the reaction according to the inventive method is performed in a proliferation system of the microorganism, the culture medium is admixed with the above mentioned organic compound as the starting material at one time or in several portions either prior to the start or during the proceeding of culturing of the microorganism. When the inventive method is performed by the reaction of resting microbiological bodies, the organic compound as the starting material is contacted with the culture solution as such taken from the culture medium after culturing of the microorganism or the bodies of the microorganism collected from the culture medium by a solid-liquid separation treatment such as centrifugation optionally followed by washing with an appropriate buffer solution and the like or crushed microbial cells which are treated in a conventional manner. When the reaction is conducted in a two-phase system or in a rare-water system, the organic solvent forming the organic phase or to dissolve a small volume of water may be one of the above named organic solvents. When the reaction is conducted in a two-phase system, the amount of the organic solvent should be sufficient to form an organic phase apart from the aqueous phase and no larger amount is required. When the reaction is conducted in a rare-water system, the amount of water added to the organic solvent should be sufficient to saturate the solvent with water or smaller. When the culture medium contains an organic solvent as one of the constituents, it can be a part of the necessary amount of the organic

4

solvent.

Culturing of the microorganism is performed aerobically at a temperature in the range from 10 to 50° C or, preferably, in the range from 20 to 40° C with a pH of the culture medium controlled in the range from 4 to 10 or, preferably, in the range from 6 to 8. The reaction is performed either aerobically or anaerobically at a temperature in the range from 5 to 90° C or, preferably, in the range from 20 to 50° C with a pH of the reaction medium controlled in the range from 2 to 11 or, preferably, in the range from 4 to 10 until the desired reaction has proceeded to a full extent.

In the following, the method of the present invention is described in more detail by way of examples preceded by a description of the microbiological process for the preparation of the transformed microorganisms used in the examples.

Microbiological Preparation 1

An LB culture medium of 10 ml volume containing 1% of bacto-tryptone, 0.5% of bacto-yeast extract and 0.5% of sodium chloride was inoculated with Pseudomonas putida PpG1064(trp⁻) containing a naphthalene-decomposing plasmid (NAH) as described in Proc. Natl. Acad. Sci. U.S.A., volume 79, page 874 (1982) and kept standing at 30° C for 4 hours to culture the microorganism. Thereafter, the culture medium was admixed under gentle agitation with 10 ml of a culture solution obtained by shaking a culture of Pseudomonas putida var. STM-603 (FERM BP-1751) in the same culture medium as above. A 4 ml portion of the mixture was taken and passed through a Millipore Filter having a pore diameter of 0.45 um to collect the microbial cells on the filter plate, which was put on an LB-agar plate culture medium containing 2% by weight of agar to culture the microorganism at 30° C for 12 hours.

After completion of culturing, the microbial cells on the filter plate were suspended in 10 ml of sterilized water and the suspension was applied to a synthetic culture medium-agar plate culture medium to conduct culturing in an atmosphere of naphthalene vapor. After 2 days of culturing, the colony formed on the culture medium was inoculated to 10 ml of a synthetic culture medium of the formulation shown below and having a pH of 7.0 with admixture of 100 mg of naphthalene. Further, 5 ml of toluene were added to the medium and culturing of the microorganism was conducted at 30° C for 24 hours so that growth of the microorganism was clearly noted.

| Formulation of the synthetic culture medium | |
|---|---|
| $NH_4Cl$ | 5 g |
| $KH_2PO_4$ | 1 g |
| $Na_2HPO_4.12H_2O$ | 3 g |
| $MgSO_4.2H_2O$ | 0.2 g |
| $CaCl_2.2H_2O$ | 0.05g |
| Deionized water | 1 liter |

On the other hand, culturing of Pseudomonas putida of the strain PpG1064(trp⁻) and Pseudomonas putida var. of the strain STM-603 was conducted in the same culture medium as above with further addition of 20 mg/liter of tryptophan to note neither of the strains could grow therein. These results clearly indicated that the strain Pseudomonas putida var. STM-603 could grow in the above mentioned culture medium with exhibition of the capacity of assimilation of naphthalene only when the strain had acquired the NAH plasmid by the conjugative transfer. It is presumable that the strains . Pseudomonas putida var. STM-603 and Pseudomonas putida PpG1064(trp⁻) could not grow under the above described conditions because of the absence of the assimilating capacity of naphthalene and resistance against toluene, respectively.

The thus obtained strain of Pseudomonas putida var. STM-603 holding the NAH plasmid is referred to hereinbelow as Pseudomonas putida var. STM-603(NAH).

Microbiological Preparation 2

An experiment of conjugative transfer was undertaken in the same manner as in Microbiological Preparation 1 described above except that the plasmid donor was Pseudomonas putida AC59 (trp⁻) carrying the camphor- and octane-decomposing plasmid (CAM-OCT) described in J. Ferment. Technol.,

volume 58 (3), page 175 (1980) and colonies of the microorganism were formed on a synthetic culture medium-agar plate culture medium in an atmosphere of camphor vapor.

The colony was inoculated to 10 ml of the same synthetic culture medium as above with further addition of 100 mg of camphor and then 5 ml of toluene were added to the medium to conduct culturing of the microorganism at 30° C for 24 hours to note that growth of the microorganism clearly took place. On the other hand, culturing of the strain Pseudomonas putida AC59 (trp⁻) and the strain Pseudomonas putida var. STM-603 was performed in the same culture medium as above with further addition of 20 mg/liter of tryptophan but neither of the strains could grow in the culture medium. This fact clearly indicated that the strain Pseudomonas putida var. STM-603 had acquired the CAM-OCT plasmid by conjugative transfer. The thus obtained strain Pseudomonas putida var. STM-603 carrying the CAM-OCT plasmid is referred to hereinbelow as Pseudomonas putida var. STM-603 (CAM-OCT).

Microbiological Preparation 3

An experiment of conjugative transfer was undertaken in the same manner as in Microbiological Preparation 1 described above by using the strain Pseudomonas putida mt-2 (ATCC 23973) carrying the toluene-decomposing plasmid (TOL) as the plasmid donor and colonies were formed on a synthetic culture medium-agar plate culture medium in an atmosphere of toluene vapor.

The colony was inoculated to 10 ml of the same synthetic culture medium as above with addition of 100 µl of toluene and culturing of the microorganism was performed with further addition of 5 ml of cyclohexane to the medium at 30° C for 24 hours to note that the microorganism was clearly grown. The thus obtained strain Pseudomonas putida var. STM-603 carrying the TOL plasmid is referred to hereinbelow as Pseudomonas putida var. STM-603 (TOL).

Microbiological Preparation 4

The strain Pseudomonas putida var. STM-603 (FERM BP-1751) was subjected to transformation by using plasmid pGR109 derived from a wide host range plasmid RSF 1010 by incorporating the gene corresponding to a benzene oxygenase and the gene corresponding to a benzene dehydrogenase described in Agric. Biol. Chem., volume 51 (6), page 1489 (1987) according to a conventional transformation method for the microorganisms belonging to the genus Pseudomonas described in Curr. Top. Microbiol. Immunol., volume 96, page 47 (1981).

A plasmid was isolated from the microbiological bodies grown in an LB culture medium with admixture of 500 µg/ml of streptomycin and it was identified to be the strain Pseudomonas putida var. STM-603 carrying the plasmid pGR109 by the electrophoretic method using an Agarose gel. The isolation of the plasmid and the electrophoresis were conducted according to a conventional procedure described, for example, in Molecular Cloning published by Cold Spring Harber Laboratiry (1982).

Microbiological Preparation 5

A 2 µg portion of plasmid pSI2074 (Japanese Patent Kokai 62-259589) derived from a vector pBR322 for Escherichia coli by incorporating the gene corresponding to a β-tyrosinase was subjected to partial cleavage at 37° C for 2 minutes using the restriction enzyme EcoRI.

On the other hand, a 2 µg portion of plasmid RSF1010 generally used as a wide host range vector was subjected to complete cleavage at 37° C for 2 hours using the restriction enzyme EcoRI followed by an alkaline phosphatase treatment in order to prevent self-cyclization of the vector.

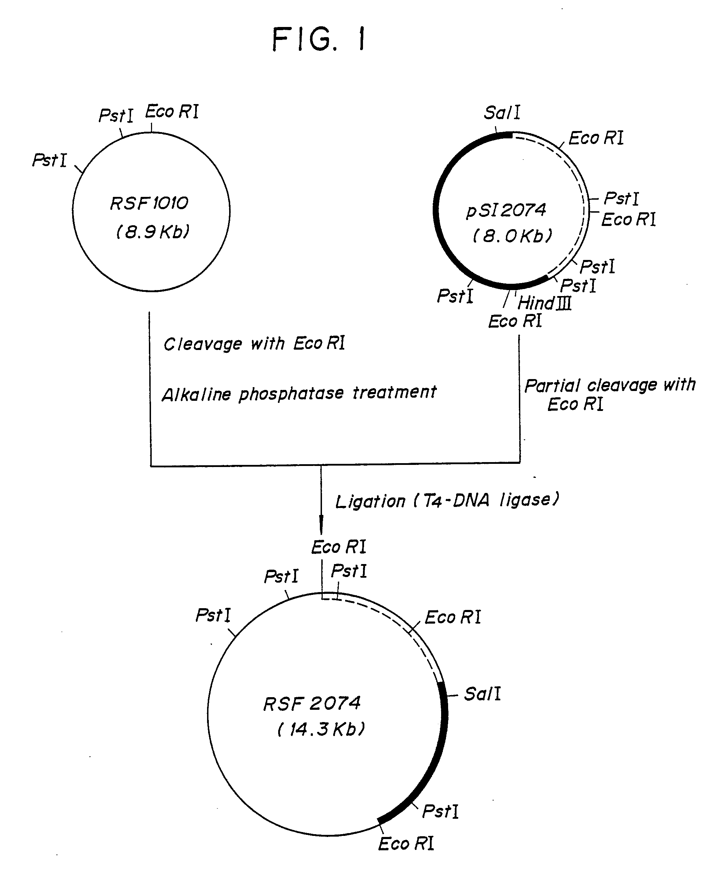

The thus obtained two kinds of DNA fragments were mixed together and subjected to a ligation reaction using T₄-DNA ligase. This ligation mixture was used for the transformation of the strain E. coli C600 and colonies were formed on an LB-agar plate culture medium containing 50 µg/liter of streptomycin and 2 mg/ml of L-tyrosine. Among these colonies, a colony was selected around which a clear zone was formed by the decomposition of L-tyrosine and it was subjected to the extraction of the plasmid DNA followed by the analysis of the restriction pattern to give a strain carrying the hybrid plasmid RSF2074 as shown in Figure 1.

The thus obtained plasmid RSF2074 was used for the transformation of the strain Pseudomonas putida var. STR-603 (FERM BP-1751) in the same manner as in Microbiological Preparation 4 to give a strain Pseudomonas putida var. STM-603 (RSF2074).

Example 1

The strain Pseudomonas putida var. STM-603 (TOL) prepared in Microbiological Preparation 3 was inoculated to 20 ml of a synthetic culture medium containing 4 g/liter of m-toluic acid as the carbon source. At the same time, 200 μl of tetradecene and 5 ml of hexane were added to the medium and culturing of the microorganism was performed at 30° C for 20 hours to effect the epoxidation reaction of the tetradecene.

The culture solution freed from the cells of the microorganism was subjected to a gas chromatographic analysis using an apparatus manufactured by Gas-chro Kogyo Co. equipped with a column of silicone GE-SE 30 and an FID detector. It was found that the hexane phase contained 80 mg/liter epoxytetradecane produced by the epoxidation reaction.

Example 2

The strain Pseudomonas putida var. STM-603 (NAH) prepared in Microbiological Preparation 1 was inoculated to 20 ml of a synthetic culture medium containing 4 g/liter of salicylic acid. At the same time, 200 μl of tetradecene and 5 ml of toluene were added to the medium and culturing of the microorganism was performed at 30° C for 20 hours to effect the epoxidation reaction of the tetradecene.

The culture solution freed from the cells of the microorganism was subjected to the same analysis as in Example 1. It was found that the toluene phase contained 50 mg/liter of epoxytetradecane produced by the epoxidation reaction.

The above result indicates that a possibility is obtained for the production of a useful organic compound under a growing condition of the microorganism in a medium admixed with an organic solvent which does not inhibit growth of the microorganism.

Example 3

The strains Pseudomonas putida var. STM-603 (NAH), Pseudomonas putida var. STM-603 (CAM-OCT) and Pseudomonas putida var. STM-603 (TOL) prepared in Microbiological Preparations 1, 2 and 3, respectively, were inoculated each to 100 ml of a syn thetic culture medium containing 4 g/liter of salicylic acid, camphor or m-toluic acid, respectively, as the carbon source and culturing thereof was performed at 30° C for 20 hours. The cells of the microorganism were collected by centrifugation of the culture medium after completion of culturing and washed with a 50 mM phosphate buffer solution having a pH of 7.0 followed by dispersion in the same buffer solution in such a concentration that the turbidity of the suspension of the microbial cells was 10 as determined by using a spectrophotometer at a wavelength of 550 nm. A 1 ml portion of the suspension was admixed with 1 ml of a 1:1 mixture of hexane and cyclohexane, 5 μl of ethyl alcohol and, as a substrate of the reaction, 10 μl of octene or tetradecene and a reaction of the resting microbial cells was performed at 30° C. After 20 hours of the reaction, the phase of the organic solvent was taken and gas-chromatographically analyzed for the amount of epoxyoctane or epoxytetradecane produced by the reaction to give the results shown in Table 1 below. These reaction products could not be detected in each of the aqueous phases.

Table 1

| Strain of P . putida var. | Product, mg/liter | |
|---|---|---|
| | Epoxyoctane | Epoxytetradecane |
| STM-603 (TOL) | 40 | 140 |
| STM-603 (NAH) | 25 | 130 |
| STM-603 (CAM-OCT) | 30 | 110 |

7

Example 4

The procedure for the reaction was substantially the same as in Example 3 using Pseudomonas putida var. STM-603 (TOL) as the microorganism strain and tetradecene as the substrate of the reaction except that the organic solvent added to the reaction mixture was toluene, xylene, ethyl benzene, dichlorobenzene, n-heptyl alcohol or n-octyl alcohol. The concentration of epoxytetradecane as the reaction product in the organic phase was 7 mg/liter in toluene, 7 mg/liter in xylene, 34 mg/liter in ethyl benzene, 10 mg/liter in dichlorobenzene, 6 mg/liter in n-heptyl alcohol and 10 mg/liter in n-octyl alcohol.

Example 5

The strain Pseudomonas putida var. STM-603 (NAH) was inoculated to 100 ml of a synthetic culture medium containing 4 g/liter of salicylic acid and cultured at 30°C for 20 hours. Cells of the microorganism were collected from the culture solution after completion of culturing and, after washing, dispersed in a 50 mM phosphate buffer solution having a pH of 7.0 in such a concentration that the turbidity of the suspension was 10 as determined using a spectrophotometer at a wavelength of 550 nm. A 1 ml portion of this suspension was admixed with 10 mg of indole, 5 µl of ethyl alcohol and 1 ml of chloroform and the reaction of resting microbial cells was performed at 30°C by shaking the mixture. After 20 hours of reaction, the reaction mixture was centrifuged and separated into precipitates of the microbial cells, an aqueous phase and a chloroform phase to find that the chloroform phase alone was colored in blue with indigo which was the reaction product from indole. The concentration of indigo in the chloroform phase was 280 mg/liter as spectrophotometrically determined by measuring the absorbance at a wavelength of 600 nm with a spectrophotometer.

Example 6

The strain Pseudomonas putida var. STM-603 (NAH) was inoculated to 100 ml of a synthetic culture medium containing 4 g/liter of succinic acid and cultured at 30°C for 24 hours. A suspension of the microbial cells was prepared in the same manner as in Example 3 and three portions of the suspension each in a volume of 1 ml were admixed with 20 mg of n-butyl acetate, 2-butyl acetate and 2-octyl acetate, respectively. After shaking the mixture at 30°C for 1 hour to effect hydrolysis of the respective acetate, the mixture was gas-chromatographically analyzed to determine the concentration of the alcohol as the hydrolysis product from the acetate ester. The results are shown in Table 2 below.

Table 2

| Alcohol produced | n-Butyl | 2-Butyl | 2-Octyl |
|---|---|---|---|
| Concentration, g/liter | 11.5 | 8.0 | 15.0 |
| % hydrolysis of ester | 89.8 | 62.5 | 99.3 |

Exampel 7

Culturing of the strain was performed in the same manner as in Example 6 and the microbial cells collected from the culture solution were suspended in a 1 ml of 2-butyl alcohol saturated with water. The suspension was admixed with 50 µl of ethyl acetate and shaken at 30°C for 24 hours to effect the ester exchange reaction between ethyl acetate and 2-butyl alcohol. The reaction mixture after completion of the reaction contained 70 mg/liter of 2-butyl acetate as determined gas-chromatographically.

Example 8

The strain Pseudomonas putida var. STM-603 (PGR109) obtained in Microbiological Preparation 4 was

8

cultured by shaking in a synthetic culture medium contianing 4 g/liter of succinic acid and 100 μg/ml of streptomycin at 30°C for 20 hours. The microbial cells collected from the culture solution by centrifugation were washed with a 50 mM phosphate buffer solution having a pH of 7.0 and dispersed in the same buffer solution in such a concentration that the turbidity of the suspension was 10 as determined using a spectrophotometer at a wavelength of 550 nm. A 1 ml portion of the suspension was admixed with 1 ml of hexane containing 5 μl of benzene to start the reaction. After 20 hours of shaking of the mixture at 30°C, the aqueous phase of the reaction mixture was analyzed by the aminoantipyrin method described in Biochem. J., volume 73, page 16 (1959) to find that the aqueous phase contained 25 mg/liter of catechol as a reaction product from benzene.

Example 9

The strain Pseudomonas putida var. STM-603 (RSF2074) obtained in Microbiological Preparation 5 was cultured in 10 ml of an LB culture medium containing 100 μg/liter of streptomycin by shaking the medium at 30°C for 20 hours. The microbial cells collected from the culture solution by centrifugation were washed with a physiological saline solution and dispersed in 10 ml of a reaction medium having a pH of 8.0 and containing 10 g/liter of pyruvic acid, 5 g/liter of ammonium acetate, 1 g/liter of EDTA and 2 g/liter of sodium sulfite. The suspension of the microbial cells in the reaction medium was further admixed with 3 ml of chloroform containing 100 mg of phenol to start the reaction at 30°C. After 2 hours of reaction, a portion of the aqueous phase was taken from the reaction mixture. The aqueous solution was put on a thin-layer chromatographic plate of silica gel to form a spot which was developed by using a 4:2:1 mixture of butyl alcohol, acetic acid and water followed by color development with a spray of a ninhydrin solution. As a result a clearly colored zone could be found showing the same $R_f$ value as a standard sample of tyrosine.

**Claims**

1. A method for conducting a microbiological reaction to produce an organic compound from a starting organic compound which comprises contacting the starting organic compound with a transformed microorganism obtained by introducing a specific gene according to a method of genetic engineering into a microorganism belonging to the genus Pseudomonas and having a resistance against aliphatic hydrocarbon compounds, alicyclic hydrocarbon compounds, aromatic hydrocarbon compounds, alcohols, ethers, ketones or derivatives thereof.

2. The method for conducting a microbiological reaction according to claim 1 wherein the microorganism belonging to the genus Pseudomonas is Pseudomonas putida.

3. The method for conducting a microbiological reaction according to claim 2 wherein the microorganism of the species Pseudomonas putida is the strain Pseudomonas putida var. STM-603 (FERM BP-1751).

4. The method for conducting a microbiological reaction according to claim 1 wherein the reaction is performed in a two-phase system composed of an aqueous phase and an organic phase or in an organic rare-water system which is an organic solvent containing a small amount of water dissolved therein.

# FIG. I